# EUROPEAN PATENT SPECIFICATION

(11) **EP 3 731 682 B1**
(45) Date of publication and mention of the grant of the patent: **14.12.2022**
(21) Application number: 18836600.9
(22) Date of filing: 28.12.2018
(51) Int. Cl.: A24D 1/22, A61M 11/04

(54) **AEROSOL-GENERATING DEVICE AND AEROSOL-GENERATING SYSTEM COMPRISING A BIMETALLIC ELEMENT**
AEROSOLERZEUGUNGSVORRICHTUNG UND AEROSOLERZEUGUNGSSYSTEM MIT EINEM BIMETALLELEMENT
DISPOSITIF DE GÉNÉRATION D'AÉROSOL ET SYSTÈME DE GÉNÉRATION D'AÉROSOL COMPRENANT UN ÉLÉMENT BIMÉTALLIQUE

(30) Priority: 29.12.2017 EP 17211112
(43) Date of publication of application: 04.11.2020
(73) Proprietor: Philip Morris Products S.A., 2000 Neuchâtel (CH)
(72) Inventor: DUC, Fabien, 2000 Neuchâtel (CH); POGET, Laurent Edouard, 2000 Neuchâtel (CH)
(74) Representative: Millburn, Julie Elizabeth
(86) International application number: PCT/EP2018/097115
(87) International publication number: WO 2019/129871

(56) References cited:
- WO-A1-2015/097005
- WO-A1-2016/156213
- DE-A1- 19 854 009
- US-A1- 2009 151 717
- US-A1- 2015 374 036

## Description

The present invention relates to an aerosol-generating device comprising a bimetallic element. In particular, the present invention relates to a non-electrical aerosol-generating device comprising a bimetallic element for generating an aerosol by the transfer of heat from a combustible heat source to a physically separate aerosol-generating substrate. The present invention also relates to an aerosol-generating system comprising the aerosol-generating device and one or more combustible heat sources.

A number of aerosol-generating articles in which tobacco material is heated rather than combusted have been proposed in the art. An aim of such 'heated' aerosol-generating articles is to reduce known harmful smoke constituents of the type produced by the combustion and pyrolytic degradation of tobacco in conventional smoking articles, such as conventional lit-end cigarettes.

Typically in heated aerosol-generating articles, an aerosol is generated by the transfer of heat from a heat source, for example, a chemical, electrical or combustible heat source, to a physically separate aerosol-generating substrate, which may be located within, around or downstream of the heat source.

In one type of heated aerosol-generating article, an aerosol is generated by the transfer of heat from a combustible carbonaceous heat source to a physically separate aerosol-generating substrate comprising tobacco material that is located downstream of the combustible carbonaceous heat source. In use, volatile compounds are released from the tobacco material by heat transfer to the aerosol-generating substrate from the combustible carbonaceous heat source and entrained in air drawn through the smoking article. As the released compounds cool, they condense to form an aerosol that is inhaled by the user.

In such heated aerosol-generating articles it is known to include one or more heat-conducting elements around at least a portion of the aerosol-generating substrate of the heated smoking article in order to provide conductive heat transfer from the combustible carbonaceous heat source to the aerosol-generating substrate to generate an aerosol. In particular, it is known to include a heat-conducting element around at least a rear portion of the combustible carbonaceous heat source and at least a front portion of the aerosol-generating substrate of the heated smoking article in order to provide conductive heat transfer from the combustible carbonaceous heat source to the aerosol-generating substrate to generate an aerosol. For example, WO 2009/022232 A2 discloses a smoking article comprising a combustible carbonaceous heat source, an aerosol-forming substrate downstream of the combustible heat source, and a heat-conducting element around and in direct contact with a rear portion of the combustible carbonaceous heat source and an adjacent front portion of the aerosol-forming substrate. In use, heat generated during combustion of the combustible carbonaceous heat source is transferred to the periphery of the front portion of the aerosol-forming substrate by conduction through the abutting downstream end of the combustible carbonaceous heat source and the heat-conducting element.

In another type of heated aerosol-generating article, an aerosol is generated by the transfer of heat from an electric heater to an aerosol-generating substrate comprising tobacco material. The aerosol-generating article comprising the aerosol-generating substrate is typically inserted into a chamber or cavity in a physically separate aerosol-generating device comprising the electric heater. The electric heater may be inserted within the heated aerosol-generating article or the electric heater may be located about the exterior of the heated aerosol-generating article. For example, EP 0 822 760 A2 discloses an electrical smoking system comprising a lighter and an article comprising a tobacco rod, said tobacco rod comprising a tubular tobacco web and a plug of tobacco disposed within said tubular tobacco web. The lighter has a plurality of resistive heater blades defining a receptacle for receiving the article, whereby the blades at least partially overlap said tobacco plug when said article is inserted into the receptacle.

WO 2015/097005 A1 discloses comprises a combustible heat source 10 having a front face 12 and an opposed rear face 14, a thermostatic bimetal valve 20, an aerosol-forming substrate 30, a transfer element 40 and a mouthpiece 50 in coaxial alignment. The thermostatic bimetal valve 20, the aerosol-forming substrate 30, transfer element 40 and mouthpiece 50 and a rear portion of the combustible heat source 10 are wrapped in an outer wrapper 70 of sheet material such as, for example, cigarette paper, of low air permeability. The combustible heat source 10 is cylindrical and comprises a central airflow channel 16 that extends from the front face 12 to the rear face 14 of the combustible heat source 10. The rear face 14 of the combustible heat source 10 is concave and a non-combustible substantially air impermeable first barrier 18 in the form of a disk of aluminium foil is provided on the rear face 14 of the combustible heat source 10. The thermostatic bimetal valve 20 is located immediately downstream of the combustible heat source 10 and is adhered to the first barrier 18 towards its radially outermost edge. The thermostatic bimetal disk has four peripheral openings 26, in the form of circular holes of approximately 0.6 mm diameter, and a central solid portion 28 which is larger than the diameter of the downstream end of the central airflow channel 16 of the combustible heat source 10. The smoking article 1 further comprises a heat-conducting element 60 of suitable material such as, for example, aluminium foil, around and in contact with a rear portion 62 of the combustible carbonaceous heat source 10 and a front portion 64 of the aerosol-forming substrate 30. During ignition of the combustible heat source 10, the thermostatic bimetal valve 20 is in a first position. In the first position, the central solid portion 28 of the disk 21 blocks the downstream end of the central airflow channel 16 to substantially prevent air from being drawn through the airflow channel 16. Thus, even if the user draws on the mouthpiece 50 during ignition of the combustible heat source 10, air is substantially prevented from being drawn into the aerosol-forming substrate 30 and being delivered to the user through the mouthpiece 50. As the combustible heat source 10 heats up, heat is transferred to the thermostatic bimetal valve 20 by conduction through the abutting rear face 14 of the combustible heat source 10 and via the heat-conducting element 60. The front portion 64 of the aerosol-forming substrate 30 is also heated by the combustible heat source 10 via the thermostatic bimetal valve 20 and the heat-conducting element 60. As heat is transferred to the valve 20, the temperature of the valve 20 rises until it reaches the threshold temperature, at which point the valve 20 snaps from the first position to a second position. In the second position, the bimetal disk 21 of the thermostatic bimetal valve 20 is convex and the central solid portion 28 of the disk 21 is spaced apart from the downstream end of the airflow channel 16 of the combustible heat source 10. In this position, the aerosol-forming substrate 30 is in fluid communication with the combustible heat source 10 via the openings 26 of the thermostatic bimetal disk 21 of the valve 20. When a user draws on the mouthpiece 50, air is drawn into the aerosol-forming substrate 30 of the smoking article 1 through the central airflow channel 16 of the combustible heat source 10 and the openings 26 of the thermostatic bimetal valve 20.

WO 2016/156213 A1 discloses an aerosol-generating system comprising an aerosol-generating device comprising a heater element, and an aerosol-generating article. The aerosol-generating article comprises a medicament source and a volatile delivery enhancing compound source. The aerosol-generating system further comprises a bimetallic strip provided in the aerosol-generating device or the aerosol-generating article, the bimetallic strip comprising a first end in thermal contact with the heater element and a second end in thermal contact with the volatile delivery enhancing compound source. The bimetallic strip is configured so that heating the first end of the bimetallic strip above a predetermined temperature results in displacement of the first end of the bimetallic strip away from the heater element. The bimetallic strip may have a helical shape, wherein the first end of the bimetallic strip forms a first end of the helix and wherein the second end of the bimetallic strip forms a second end of the helix.

US 2015/0374036 A1 discloses a non-combustion-type flavor inhaler provided with a heat source and a holding member for detachably holding the heat source. The heat source comprises a latent heat storage material containing a sugar alcohol of four or more carbons.

DE 198 54 009 A1 discloses a system for supplying an inhalable aerosol containing an inhaling device which has a rod-shaped housing with a rear, mouth-end area and a front area. A substrate holder, which can be heated by a heating device, is arranged in the front area of the housing and is accessible via a loading opening that is preferably located at the front end of the housing. A flow channel leads from the substrate holder to the rear end of the housing. The heating device is configured to generate heat by thermal or catalytic combustion. A substrate portion, which is configured for insertion into the substrate holder through the loading opening, contains aerosol-forming material inside a casing which has an air inlet and an aerosol outlet.

In heated aerosol-generating articles in which an aerosol-generating substrate is heated rather than combusted, the temperature attained in the aerosol-generating substrate has a significant impact on the ability to generate a sensorially acceptable aerosol. It is typically desirable to maintain the temperature of the aerosol-generating substrate within a certain range in order to optimise the aerosol delivery to the user. If the temperature of the aerosol-forming substrate drops too low, for instance, it may adversely impact the consistency and the amount of aerosol delivered to the user. However, if the temperature of the aerosol-generating substrate becomes too high it may adversely result in combustion or pyrolysis of the aerosol-generating substrate.

According to the invention there is provided aerosol-generating device comprising: a housing comprising a first tubular section for receiving a combustible heat source and a second tubular section for receiving an aerosol-generating substrate; a heat-conductive element disposed between the first tubular section and the second tubular section of the housing for transferring heat from a combustible heat source received in the first tubular section to an aerosol-generating substrate received in the second tubular section, the heat-conductive element comprising a bimetallic element; and a heat-insulative sleeve around at least a portion of the second tubular section of the housing, wherein the heat-insulative sleeve comprises one or more air inlets, wherein the bimetallic element is arranged to deform from a first position, in which a first portion of the bimetallic element is proximate the first tubular section of the housing and a second portion of the bimetallic element is proximate the second tubular section of the housing, to a second position, in which the first portion of the bimetallic element is displaced away from the first tubular section of the housing towards the second tubular section of the housing, when the bimetallic element is heated above a threshold temperature.

According to the invention there is also provided an aerosol-generating system comprising: an aerosol-generating device according to the invention; and one or more combustible heat sources for insertion into the first tubular section of the aerosol-generating device.

As used herein, the term aerosol-generating device' is used to describe a device that interacts with an aerosol-generating substrate to generate an aerosol that is directly inhalable into a user's lungs through the user's mouth.

As used herein, the term aerosol-generating substrate' is used to describe a substrate comprising aerosol-forming material capable of releasing upon heating volatile compounds, which can form an aerosol. The aerosols generated from aerosol-generating substrates for use with the aerosol-generating device may be visible or invisible and may include vapours (for example, fine particles of substances, which are in a gaseous state, that are ordinarily liquid or solid at room temperature) as well as gases and liquid droplets of condensed vapours.

Provision of a heat-conductive element comprising a bimetallic element between the first tubular section and the second tubular section of the housing of the aerosol-generating device advantageously allows control of conductive heat transfer from a combustible heat source received in the first tubular section of the housing to an aerosol-generating substrate received in the second tubular section of the housing via the heat-conductive element. As described further below, the aerosol-generating device thereby advantageously allows control of the temperature to which the aerosol-generating substrate is heated. In particular, the aerosol-generating device thereby advantageously allows self-regulating control of the maximum temperature to which an aerosol-generating substrate received in the second tubular section of the housing is heated.

The aerosol-generating device is reusable.

The first tubular section of the housing of the aerosol-generating device is for removably receiving a combustible heat source. That is, the first tubular section of the housing of the aerosol-generating device is configured to removably receive a combustible heat source.

Initially, the first tubular section of the housing of the aerosol-generating device is empty. A combustible heat source may be inserted into the empty first tubular section of the housing. Once the combustible heat source has been consumed, the combustible heat source may be removed from the first tubular section of the housing. To reuse the aerosol-generating device, a further combustible heat source may subsequently be inserted into the empty first tubular section of the housing.

The aerosol-generating system according to the invention comprises: a reusable aerosol-generating device according to the invention; and one or more combustible heat sources for insertion into the first tubular section of the reusable aerosol-generating device.

The second tubular section of the housing of the aerosol-generating device is for removably receiving an aerosol-generating substrate. That is, the second tubular section of the housing of the aerosol-generating device is configured to removably receive an aerosol-generating substrate.

Initially, the second tubular section of the housing of the aerosol-generating device is empty. An aerosol-generating substrate may be inserted into the empty second tubular section of the housing. Once the aerosol-generating substrate has been consumed, the aerosol-generating substrate may be removed from the second tubular section of the housing. To reuse the aerosol-generating device, a further aerosol-generating substrate may subsequently be inserted into the empty second tubular section of the housing.

The first tubular section and the second tubular section of the housing may be formed from the same or different materials.

The first tubular section of the housing may be formed from one or more suitable materials that are substantially thermally stable and non-combustible at temperatures reached during ignition and combustion of a combustible heat source received in the first tubular section.

The second tubular section of the housing may be formed from one or more suitable materials that are substantially thermally stable and non-combustible at temperatures to which an aerosol-generating substrate received in the second tubular section of the housing is heated during use of the aerosol-generating device.

Preferably, one or both of the first tubular section and the second tubular section of the housing comprise thermally-conductive material. More preferably, both the first tubular section and the second tubular section of the housing comprise thermally-conductive material. This advantageously allows for conductive heat transfer from a combustible heat source received in the first tubular section of the housing to the periphery of an aerosol-generating substrate received in the second tubular section of the housing via the first tubular section and the second tubular section of the housing.

In certain embodiments, one or both of the first tubular section and the second tubular section of the housing are formed of thermally-conductive material.

In certain embodiments, both of the first tubular section and the second tubular section of the housing are formed of thermally-conductive material.

The first tubular section and the second tubular section of the housing may be partially formed of thermally-conductive material or entirely formed of thermally-conductive material.

Suitable materials from which the first tubular section and the second tubular section of the housing may be formed are known in the art and include, but are not limited to, aluminium, steel, graphite and combinations thereof.

The first tubular section of the housing may comprise one or more air inlets to allow air to be drawn into the first tubular section of the housing. This may advantageously facilitate ignition and sustained combustion of a combustible heat source received in the first tubular section of the housing. The one or more air inlets in the first tubular section of the housing may advantageously allow combustion and decomposition products and other materials formed during ignition and combustion of a combustible heat source received in the first tubular section of the housing to be exhausted from the first tubular section of the housing.

The second tubular section of the housing may comprise one or more air inlets to allow air to be drawn into the second tubular section of the housing. This may advantageously facilitate the drawing of air through an aerosol-generating substrate received in the second tubular section of the housing for inhalation by a user.

Preferably, the housing and the heat-conductive element are configured such that airflow from the first tubular section to the second tubular section of the housing is substantially prevented when the bimetallic element is in the first position and when the bimetallic element is in the second position. This may advantageously substantially prevent or inhibit combustion and decomposition products and other materials formed during ignition and combustion of a combustible heat source received in the first tubular section of the housing from entering air drawn through an aerosol-generating article received in the second tubular section of the housing for inhalation by a user. This may also advantageously substantially prevent or inhibit activation of combustion of a combustible heat source received in the first tubular section of the housing during puffing by a user, which may assist in substantially preventing or inhibiting spikes in the temperature of the aerosol-forming substrate during puffing by a user.

The heat-conductive element disposed between the first tubular section and the second tubular section of the housing comprises a bimetallic element that undergoes a mechanical displacement in response to a temperature change.

As used herein, the term 'bimetallic element' is used to describe an element comprising two metallic layers having different coefficients of thermal expansion. The two metallic layers may be joined together by any suitable known process. For example, the two metallic layers may be joined together by cladding or welding. Suitable metals and alloys for forming the two metallic layers of the bimetallic element include, but are not limited to, aluminium, aluminium alloys, beryllium alloys, bismuth, brass, bronze, cadmium, cobalt alloys, copper, copper-nickel alloys, gold, iron, lead, magnesium, magnesium alloys, monel, nickel, nickel alloys, nickel aluminides, niobium, niobium alloys, palladium, platinum, rhenium, silver, silver alloys, steel, tantalum, tantalum alloys, tin, tin alloys, titanium, titanium aluminides, uranium, vanadium alloys, zinc, zinc alloys, and zirconium.

Suitable commercially available bimetallic materials from which the bimetallic element may be formed include, but are not limited to, KANTHAL 60, KANTHAL 180R05, KANTHAL 145R10, KANTHAL 135R05, KANTHAL 130R03, KANTHAL 127R09 and KANTHAL 115R09 available from Kanthal AB, Sweden.

The bimetallic element is arranged to deform from a first position, in which a first portion of the bimetallic element is proximate the first tubular section of the housing and a second portion of the bimetallic element is proximate the second tubular section of the housing, to a second position, in which the first portion of the bimetallic element is displaced away from the first tubular section of the housing towards the second tubular section of the housing, when the bimetallic element is heated above a threshold temperature.

As used herein, the term 'deform' is used to describe one or both of a change of shape or a change of dimensions of a bimetallic element.

The bimetallic element is preferably arranged to deform from the first position to the second position when heated to above a threshold temperature of at least about 200°C, more preferably when heated to above a threshold temperature of at least at least about 300°C, most preferably when heated to above a threshold temperature of at least about 350°C.

The bimetallic element elastically deforms from the first position to the second position when the bimetallic element is heated above the threshold temperature.

The bimetallic element is arranged to deform from the second position to the first position when the bimetallic element cools below the threshold temperature.

The bimetallic element elastically deforms from the second position to the first position when the bimetallic element cools below the threshold temperature.

In certain embodiments, the bimetallic element may be pre-stressed such that it deforms from the first position to the second position with a snap action. In such embodiments, the bimetallic element instantaneously deforms from the first position to the second position when heated above the threshold temperature.

In certain embodiments, the bimetallic element may not be pre-stressed. In such embodiments, the bimetallic element gradually deforms from the first position to the second position as the temperature of the bimetallic element increases.

In certain embodiments, the bimetallic element may bend from the first position to the second position when heated above the threshold temperature.

In certain embodiments, the bimetallic element may be substantially flat in the first position and concavely curved relative to the first tubular section in the second position.

In certain embodiments, the bimetallic element may be convexly curved relative to the first tubular section in the first position and substantially flat in the second position.

In certain embodiments, the bimetallic element may be convexly curved relative to the first tubular section in the first position and concavely curved relative to the first tubular section in the second position.

In certain embodiments, the bimetallic element may contract from the first position to the second position when heated above the threshold temperature.

The bimetallic element may have any suitable shape. For example, the bimetallic element may be a bimetallic strip, a bimetallic disk or a bimetallic coil, for example a bimetallic helical coil or a bimetallic spiral coil.

Where the bimetallic element is a bimetallic strip or a bimetallic disk, the first portion of the bimetallic element may be a first metallic layer of the bimetallic element and the second portion of the bimetallic element may be a second metallic layer of the bimetallic element.

Where the bimetallic element is a bimetallic strip or a bimetallic disk, the first portion of the bimetallic element may be a first metallic layer of the bimetallic element and the second portion of the bimetallic element may be a second metallic layer of the bimetallic element.

Where the bimetallic element is a bimetallic coil, the first portion of the bimetallic element may be a first end of the bimetallic element and the second portion of the bimetallic element may be a second end of the bimetallic element.

In certain embodiments, the second portion of the bimetallic element may be fixed in position relative to the first tubular section and the second tubular section of the housing.

During use of the aerosol-generating device, heat is transferred from a combustible heat source received in the first tubular section of the housing to an aerosol-generating substrate received in the second tubular section of the housing by conduction via the heat-conductive element comprising the bimetallic element.

To facilitate conductive heat transfer from a combustible heat source received in the first tubular section of the housing to an aerosol-generating substrate received in the second tubular section, the bimetallic element advantageously has a thermal conductivity of between about 1 watts per metre Celsius (W/(m°C)) and about 400 watts per metre Celsius (W/(m°C)) as measured in accordance with ASTM C518-10 at ambient temperature, for example at 20 degrees Celsius.

Preferably, the bimetallic element has a thermal conductivity of at least about 20 watts per metre Celsius (W/(m°C)), more preferably a thermal conductivity of at least about 40 watts per metre Celsius (W/(m°C)) as measured in accordance with ASTM C518-10 at ambient temperature, for example at 20 degrees Celsius.

To increase the thermal conductivity thereof, the bimetallic element may comprise two metallic layers having different coefficients of thermal expansion and an intermediate layer of material having a high thermal conductivity, such as, for example, copper, between the two metallic layers having different coefficients of thermal expansion.

The composition, shape and dimensions of the bimetallic element may be selected to provide a desired speed of deformation, a desired degree of deformation and a desired thermal transfer capacity of the bimetallic element.

During use of the aerosol-generating device, in the first position, in which the first portion of the bimetallic element is proximate the first tubular section of the housing and the second portion of the bimetallic element is proximate the second tubular section of the housing, the bimetallic element is heated by a combustible heat source received in the first tubular section of the housing. When the bimetallic element is heated above the threshold temperature, the bimetallic element automatically deforms from the first position to the second position, in which the first portion of the bimetallic element is displaced away from the first tubular section of the housing towards the second tubular section of the housing.

In the second position, heat transfers by conduction from the heat-conductive element comprising the bimetallic element to an aerosol-generating substrate received in the second tubular section of the housing and the bimetallic element cools. When the bimetallic element cools below the threshold temperature, the bimetallic element automatically deforms from the second position to the first position and the first portion of the bimetallic element is displaced away from the second tubular section of the housing towards the first tubular section of the housing to facilitate heat transfer from the combustible heat source to the bimetallic element.

Deformation of the bimetallic element between the first position and the second position provides thermostatic control of the temperature of an aerosol-generating substrate received in the second tubular section of the housing through dynamic adjustment of conductive heat transfer from a combustible heat source received in the first tubular section of the housing to the aerosol-generating substrate via the heat-conductive element comprising the bimetallic element.

In certain embodiments, the bimetallic element may control conductive heat transfer from a combustible heat source received in the first tubular section of the housing to an aerosol-generating substrate received in the second tubular section of the housing via the heat-conductive element so that the temperature of the aerosol-generating article is between about 200°C and about 350°C.

In the first position, the first portion of the bimetallic element is advantageously in thermal contact with a combustible heat source received in the first tubular section of the housing.

In the first position, the first portion of the bimetallic element may be in direct thermal contact with a combustible heat source received in the first tubular section of the housing.

In the first position, the first portion of the bimetallic element may be in indirect thermal contact with a combustible heat source received in the first tubular section of the housing.

As used herein, the term 'thermal contact' is used to describe two components that are arranged so that heat can be transferred between the two components by conduction. The two components may contact each other directly. As used herein, this is described as 'direct thermal contact'. One or more additional thermally-conductive components may be provided between the two components so that heat can be transferred between the two components by conduction via the one or more additional thermally-conductive components. As used herein, this is described as 'indirect thermal contact'.

In certain embodiments, the heat-conductive element may comprise a first heat-conductive component disposed between the bimetallic element and the first tubular section of the housing.

The first heat-conductive component may be in direct thermal contact with a combustible heat source received in the first tubular section of the housing.

In the first position, the first portion of the bimetallic element may be in indirect thermal contact with a combustible heat source received in the first tubular section of the housing via the first heat-conductive component.

The first heat-conductive component may comprise a first elongate heat-conductive member that projects into the first tubular section of the housing for insertion into a combustible heat source received in the first tubular section.

The first elongate heat-conductive member may advantageously facilitate conductive heat transfer from a combustible heat source received in the first tubular section of the housing to an aerosol-generating article received in the second tubular section of the housing via the heat-conductive element.

The first elongate heat-conductive member may advantageously facilitate retention of a combustible heat source in the first tubular section of the housing.

In the second position, the second portion of the bimetallic element is advantageously in thermal contact with an aerosol-generating agent received in the second tubular section of the housing.

In the second position, the second portion of the bimetallic element may be in direct thermal contact with an aerosol-generating agent received in the second tubular section of the housing.

In the second position, the second portion of the bimetallic element may be in indirect thermal contact with an aerosol-generating agent received in the second tubular section of the housing.

In the first position, the second portion of the bimetallic element may be in thermal contact with an aerosol-generating agent received in the second tubular section of the housing.

In the first position, the second portion of the bimetallic element may be in direct thermal contact with an aerosol-generating agent received in the second tubular section of the housing.

In the first position, the second portion of the bimetallic element may be in indirect thermal contact with an aerosol-generating agent received in the second tubular section of the housing.

In certain embodiments, the heat-conductive element may comprise a second heat-conductive component disposed between the bimetallic element and the second tubular section of the housing.

The second heat-conductive component may be in direct thermal contact with an aerosol-generating article received in the second tubular section of the housing.

In the second position, the second portion of the bimetallic element may be in indirect thermal contact with an aerosol-generating article received in the second tubular section of the housing via the second heat-conductive component.

In the first position, the second portion of the bimetallic element may be in indirect thermal contact with an aerosol-generating article received in the second tubular section of the housing via the second heat-conductive component.

The second heat-conductive component may comprise a second elongate heat-conductive member that projects into the second tubular section of the housing for insertion into an aerosol-generating substrate received in the second tubular section.

The second elongate heat-conductive member may advantageously facilitate conductive heat transfer from a combustible heat source received in the first tubular section of the housing to an aerosol-generating substrate received in the second tubular section of the housing via the heat-conductive element.

The second elongate heat-conductive member advantageously facilitate retention of an aerosol-generating substrate in the second tubular section of the housing.

The aerosol-generating device comprises a heat-insulative sleeve around at least a portion of the second tubular section of the housing. The heat-insulative sleeve may advantageously reduce heat loss from the aerosol-generating device. The heat-insulative sleeve may advantageously facilitate handling of the aerosol-generating device by a user.

The heat-insulative sleeve is around at least a portion of the second tubular section. The heat-insulative sleeve may be around at least a portion of the second tubular section and at least a portion of the first tubular section of the housing.

The heat-insulative sleeve may be around substantially the entire length of the second portion of the housing.

In certain embodiments, the heat-insulative sleeve may be around substantially the entire length of the first portion of the housing.

The heat-insulative sleeve comprises one or more air inlets to allow air to be drawn into the aerosol-generating device.

The one or more air inlets in the heat-insulative sleeve may allow air to be drawn into the first tubular section of the housing. This may advantageously facilitate ignition and sustained combustion of a combustible heat source received in the first tubular section of the housing.

The one or more air inlets in the heat-insulative sleeve may allow air to be drawn into the second tubular section of the housing. This may advantageously facilitate the drawing of air through an aerosol-generating substrate received in the second tubular section of the housing for inhalation by a user.

The one or more air inlets in the heat-insulative sleeve may advantageously allow combustion and decomposition products and other materials formed during ignition and combustion of a combustible heat source received in the first tubular section of the housing to be exhausted from the aerosol-generating device.

The aerosol-generating device may comprise a heat-insulative cover around at least a portion of the first tubular section that is removably connected to the heat-insulative sleeve.

The heat-insulative cover may project axially outward beyond the first tubular section of the housing. This may advantageously improve shielding of a user from a combustible heat source received in the first tubular section of the housing during use of the aerosol-generating device.

Prior to use of the aerosol-generating device, the heat-insulative cover may advantageously be disconnected from the heat-insulative sleeve to facilitate insertion of a combustible heat source into the first tubular section of the housing.

After use of the aerosol-generating device, the heat-insulative cover may advantageously be disconnected from the heat-insulative sleeve to facilitate removal of a combustible heat source from the first tubular section of the housing.

Preferably, the combustible heat source is a solid combustible heat source.

More preferably, the combustible heat source is a monolithic solid combustible heat source. That is, a one-piece solid combustible heat source.

Advantageously, the combustible heat source is substantially cylindrical.

The combustible heat source may have a length of between about 7 millimetres and about 17 millimetres, for example a length of between about 7 millimetres and about 15 millimetres or a length of between about 7 millimetres and about 13 millimetres.

The combustible heat source may have a diameter of between about 5 millimetres and about 9 millimetres, for example a diameter of between about 7 millimetres and about 8 millimetres.

Advantageously, the combustible heat source is a combustible carbonaceous heat source.

As used herein, the term 'carbonaceous' is used to describe a combustible heat source comprising carbon.

Advantageously, the combustible heat source comprises carbonised material.

Advantageously, the combustible carbonaceous heat source has a carbon content of at least about 35 percent by dry weight of the combustible carbonaceous heat source.

The combustible carbonaceous heat source may have a carbon content of at least about 40 percent by dry weight of the combustible carbonaceous heat source or a carbon content of at least about 45 percent by dry weight of the combustible carbonaceous heat source.

The combustible carbonaceous heat source may be a combustible carbon-based heat source.

As used herein, the term 'carbon-based' is used to describe a combustible carbonaceous heat source comprised primarily of carbon, that is a combustible carbonaceous heat source having a carbon content of at least about 50 percent by dry weight of the combustible carbonaceous heat source. For example, the combustible carbonaceous heat source may have a carbon content of at least about 60 percent by dry weight of the combustible carbonaceous heat source or at least about 70 percent by dry weight of the combustible carbonaceous heat source or at least about 80 percent by dry weight of the combustible carbonaceous heat source.

The combustible carbonaceous heat source may be formed from one or more suitable carbon-containing materials.

One or more binders may be combined with the one or more carbon-containing materials. In such embodiments, the combustible carbonaceous heat source may comprise one or more organic binders, one or more inorganic binders or a combination of one or more organic binders and one or more inorganic binders.

The combustible carbonaceous heat source may comprise one or more additives in order to improve the properties of the combustible carbonaceous heat source. Suitable additives include, but are not limited to: additives to promote consolidation of the combustible carbonaceous heat source (for example, sintering aids); additives to promote ignition of the combustible carbonaceous heat source (for example, oxidisers such as perchlorates, chlorates, nitrates, peroxides, permanganates, zirconium and combinations thereof); additives to promote combustion of the combustible carbonaceous heat source (for example, potassium and potassium salts, such as potassium citrate); additives to promote decomposition of one or more gases produced by combustion of the combustible carbonaceous heat source (for example catalysts, such as CuO, Fe₂O₃ and Al₂O₃); or any combination thereof.

Advantageously, the combustible carbonaceous heat source comprises at least one ignition aid. In certain preferred embodiments, the combustible carbonaceous heat source comprises at least one ignition aid as described in WO 2012/164077 A1.

Suitable processes for producing combustible carbonaceous heat sources for use with the aerosol-generating device are known in the art and include, but are not limited to, pressing processes and an extrusion processes.

In certain preferred embodiments, the combustible heat source is a pressed combustible carbonaceous heat source.

The combustible heat source may be a non-blind combustible heat source.

As used herein, the term 'non-blind' is used to describe a combustible heat source including at least one airflow channel extending along the length of the combustible heat source.

As used herein, the term 'airflow channel' is used to describe a channel extending along the length of a combustible heat source through which air may be drawn.

Advantageously, the combustible heat source is a blind combustible heat source.

As used herein, the term 'blind' is used to describe a combustible heat source that does not include any airflow channels extending along the length of the combustible heat source through which air may be drawn.

Where the combustible heat source is a non-blind combustible heat source or a blind combustible heat source, the combustible heat source may comprise one or more closed or blocked passageways through which air may not be drawn.

For example, the combustible heat source may comprise one or more closed passageways that extend only part way along the length of the combustible heat source.

The inclusion of one or more closed air passageways increases the surface area of the combustible heat source that is exposed to oxygen from the air and may advantageously facilitate ignition and sustained combustion of the combustible heat source.

Where the heat-conductive element of the aerosol-generating device comprises a first elongate heat-conductive member that projects into the first tubular section of the housing, the combustible heat source advantageously comprises a cavity into which the first elongate heat-conductive member may be inserted.

Advantageously, the housing comprises a first tubular section for receiving a combustible heat source and a second tubular section for receiving an aerosol-generating article comprising an aerosol-generating substrate.

The aerosol-generating device may advantageously allow aerosol-generating articles intended for use with an aerosol-generating device comprising an electric heater to instead be used with a combustible heat source.

Advantageously, the aerosol-generating substrate comprises aerosol-forming material comprising an aerosol-former.

The aerosol former may be any suitable compound or mixture of compounds that, in use, facilitates formation of a dense and stable aerosol and that is substantially resistant to thermal degradation at the operating temperature of the aerosol-generating article. Suitable aerosol formers are known in the art and include, but are not limited to: polyhydric alcohols, such as triethylene glycol, propylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate.

Advantageously, the aerosol former comprises one or more polyhydric alcohols.

More advantageously, the aerosol former comprises glycerine.

Preferably, the aerosol-generating substrate is a solid aerosol-forming substrate. The aerosol-generating substrate may comprise both solid and liquid components.

The aerosol-generating substrate may comprise plant-based material. The aerosol-generating substrate may comprise homogenised plant-based material.

The aerosol-generating substrate may comprise nicotine.

The aerosol-generating substrate may comprise tobacco material.

As used herein, the term 'tobacco material' is used to describe any material comprising tobacco, including, but not limited to, tobacco leaf, tobacco rib, tobacco stem, tobacco stalk, tobacco dust, expanded tobacco, reconstituted tobacco material and homogenised tobacco material.

The tobacco material may, for example, be in the form of powder, granules, pellets, shreds, strands, strips, sheets or any combination thereof.

Advantageously, the aerosol-generating substrate comprises homogenised tobacco material.

As used herein, the term 'homogenised tobacco material' is used to describe a material formed by agglomerating particulate tobacco.

In certain embodiments, the aerosol-generating substrate advantageously comprises a plurality of strands of homogenised tobacco material.

Advantageously, the plurality of strands of homogenised tobacco material may be aligned substantially parallel to one another within the aerosol-generating substrate.

In certain embodiments, the aerosol-generating substrate advantageously comprises a gathered sheet of homogenised tobacco material.

The aerosol-generating substrate may comprise a rod comprising a gathered sheet of homogenised tobacco material.

The aerosol-generating substrate may comprise aerosol-forming material and a wrapper around and in contact with the aerosol-forming material.

The wrapper may be formed from any suitable sheet material that is capable of being wrapped around aerosol-forming material to form an aerosol-generating substrate.

In certain embodiments, the aerosol-generating substrate may comprise a rod comprising a gathered sheet of homogenised tobacco material and a wrapper around and in contact with the tobacco material.

As used herein, the term 'rod' is used to denote a generally cylindrical element of substantially circular, oval or elliptical cross-section.

As used herein, the term 'sheet' is used to describe a laminar element having a width and length substantially greater than the thickness thereof.

As used herein, the term 'gathered' is used to describe a sheet that is convoluted, folded, or otherwise compressed or constricted substantially transversely to the longitudinal axis of the aerosol-generating article.

In certain embodiments, the aerosol-generating substrate advantageously comprises a gathered textured sheet of homogenised tobacco material.

As used herein, the term 'textured sheet' is used to describe a sheet that has been crimped, embossed, debossed, perforated or otherwise deformed.

Use of a textured sheet of homogenised tobacco material may advantageously facilitate gathering of the sheet of homogenised tobacco material to form the aerosol-forming substrate.

The aerosol-generating substrate may comprise a gathered textured sheet of homogenised tobacco material comprising a plurality of spaced-apart indentations, protrusions, perforations or any combination thereof.

The aerosol-generating substrate may comprise a gathered crimped sheet of homogenised tobacco material.

As used herein, the term 'crimped sheet' is used to describe a sheet having a plurality of substantially parallel ridges or corrugations.

Advantageously, when an aerosol-generating article comprising the aerosol-generating substrate has been assembled, the substantially parallel ridges or corrugations extend along or parallel to the longitudinal axis of the aerosol-generating article. This facilitates gathering of the crimped sheet of homogenised tobacco material to form the aerosol-generating substrate.

However, it will be appreciated that crimped sheets of homogenised tobacco material for inclusion in aerosol-forming substrates of aerosol-generating articles for use with the aerosol-generating device may alternatively or in addition have a plurality of substantially parallel ridges or corrugations that are disposed at an acute or obtuse angle to the longitudinal axis of the aerosol-generating article when the aerosol-generating article has been assembled.

Preferably, the aerosol-generating substrate is substantially cylindrical.

The aerosol-generating substrate may have a length of between about 5 millimetres and about 20 millimetres, for example a length of between about 6 millimetres and about 15 millimetres or a length of between about 7 millimetres and about 12 millimetres.

The aerosol-generating substrate may have a diameter of between about 5 millimetres and about 9 millimetres, for example a diameter of between about 7 millimetres and about 8 millimetres.

Aerosol-generating articles for use with the aerosol-generating device may comprise an aerosol-generating substrate and one or more other components.

The aerosol-generating substrate and one or more other components may be assembled within one or more wrappers of the aerosol-generating article to form an elongate rod having a proximal end and an opposed distal end. Aerosol-generating articles for use with the aerosol-generating device may thus resemble conventional lit-end cigarettes.

The one or more other components may comprise one or more of a support element, a transfer or spacer element, an aerosol-cooling element and a mouthpiece.

Aerosol-generating articles for use with the aerosol-generating device may comprise a support element immediately downstream of the aerosol-generating substrate.

The support element may be configured to resist downstream movement of the aerosol-forming substrate within the aerosol-generating article.

The support element may comprise at least one open-ended tubular hollow body.

Aerosol-generating articles for use with the aerosol-generating device may comprise a transfer element or spacer element downstream of the aerosol-generating substrate. That is, a transfer element or spacer element located between the aerosol-generating substrate and the proximal end of the aerosol-generating article.

The transfer element may abut the aerosol-generating substrate. Alternatively, the transfer element may be longitudinally spaced apart from the aerosol-generating substrate.

The inclusion of a transfer element advantageously allows cooling of the aerosol generated by heat transfer to the aerosol-generating substrate. The inclusion of a transfer element also advantageously allows the overall length of aerosol-generating articles for use with the aerosol-generating device to be adjusted to a desired value, for example to a length similar to that of a conventional cigarette, through an appropriate choice of the length of the transfer element.

The transfer element may have a length of between about 7 millimetres and about 50 millimetres, for example a length of between about 10 millimetres and about 45 millimetres or a length of between about 15 millimetres and about 30 millimetres. The transfer element may have other lengths depending upon the desired overall length of the aerosol-generating article, and the presence and length of other components within the aerosol-generating article.

The transfer element may comprise at least one open-ended tubular hollow body. In such embodiments, in use, air drawn into the aerosol-generating article passes through the at least one open-ended tubular hollow body as it passes downstream through the aerosol-generating article from the aerosol-generating substrate to the proximal end of the aerosol-generating article.

The transfer element may comprise at least one open-ended tubular hollow body formed from one or more suitable materials that are substantially thermally stable at the temperature of the aerosol generated by the transfer of heat to the aerosol-generating substrate. Suitable materials are known in the art and include, but are not limited to, paper, cardboard, plastics, such a cellulose acetate, ceramics and combinations thereof.

Aerosol-generating articles for use with the aerosol-generating device may comprise an aerosol-cooling element or heat exchanger downstream of the aerosol-generating substrate. That is, an aerosol-cooling element or heat exchanger located between the aerosol-generating substrate and the proximal end of the aerosol-generating article.

The aerosol-cooling element may comprise a plurality of longitudinally extending channels.

The aerosol-cooling element may comprise a gathered sheet of material selected from the group consisting of metallic foil, polymeric material, and substantially non-porous paper or cardboard. In certain embodiments, the aerosol-cooling element may comprise a gathered sheet of material selected from the group consisting of polyethylene (PE), polypropylene (PP), polyvinylchloride (PVC), polyethylene terephthalate (PET), polylactic acid (PLA), cellulose acetate (CA), and aluminium foil.

The aerosol-cooling element may comprise a gathered sheet of biodegradable polymeric material, such as polylactic acid (PLA) or a grade of Mater-Bi^{®} (a commercially available family of starch based copolyesters).

Where aerosol-generating articles for use with the aerosol-generating device comprise a transfer element downstream of the aerosol-generating substrate and an aerosol-cooling element downstream of the aerosol-generating substrate, the aerosol-cooling element is preferably downstream of the transfer element. That is, the aerosol-cooling element is preferably located between the transfer element and the proximal end of the aerosol-generating article.

Aerosol-generating articles for use with the aerosol-generating device may comprise a mouthpiece downstream of the aerosol-generating substrate. That is, a mouthpiece located between the aerosol-generating substrate and the proximal end of the aerosol-generating article.

Preferably, the mouthpiece is located at the proximal end of the aerosol-generating article.

Preferably, the mouthpiece is of low filtration efficiency, more preferably of very low filtration efficiency.

The mouthpiece may be a single segment or component mouthpiece.

Alternatively, the mouthpiece may be a multi-segment or multi-component mouthpiece.

The mouthpiece may comprise a filter comprising one or more segments comprising suitable filtration materials. Suitable filtration materials are known in the art and include, but are not limited to, cellulose acetate and paper. Alternatively or in addition, the mouthpiece may comprise one or more segments comprising absorbents, adsorbents, flavourants, and other aerosol modifiers and additives or combinations thereof.

Aerosol-generating articles for use with the aerosol-generating device may comprise one or more aerosol modifying agents downstream of the aerosol-generating substrate. For example, where included, one or more of the mouthpiece, transfer element and aerosol-cooling element of aerosol-generating articles for use with the aerosol-generating device may comprise one or more aerosol modifying agents.

As used herein, the term 'aerosol-modifying agent' is used to describe any agent that, in use, modifies one or more features or properties of an aerosol generated by the aerosol-generating substrate of the aerosol-generating article.

Suitable aerosol-modifying agents include, but are not limited to: flavourants; and chemesthetic agents.

As used herein, the term 'chemesthetic agent' is used to describe any agent that, in use, is perceived in the oral or olfactory cavities of a user by means other than, or in addition to, perception via taste receptor or olfactory receptor cells. Perception of chemesthetic agents is typically via a "trigeminal response," either via the trigeminal nerve, glossopharyngeal nerve, the vagus nerve, or some combination of these. Typically, chemesthetic agents are perceived as hot, spicy, cooling, or soothing sensations.

Aerosol-generating articles for use with the aerosol-generating device may comprise one or more aerosol modifying agents that are both a flavourant and a chemesthetic agent downstream of the aerosol-generating substrate. For example, where included, one or more of the mouthpiece, transfer element and aerosol-cooling element of aerosol-generating articles for use with the aerosol-generating device may comprise menthol or another flavourant that provides a cooling chemesthetic effect.

Where aerosol-generating articles for use with the aerosol-generating device comprise one or more components downstream of the aerosol-generating substrate such as, for example, a transfer element, a cooling element or a mouthpiece, the second tubular section of the housing of the aerosol-generating device may receive some or all of the components of the aerosol-generating article downstream of the aerosol-generating substrate.

Aerosol-generating articles for use with the aerosol-generating device may have an external diameter that is substantially the same as an internal diameter of the second tubular section of the housing of the aerosol-generating device. This may advantageously facilitate retention of the aerosol-generating articles in the second tubular section of the housing.

Aerosol-generating articles for use with the aerosol-generating device may have an external diameter that is smaller than an internal diameter of the second tubular section of the housing of the aerosol-generating device. This may advantageously facilitate the drawing of air through the aerosol-generating substrates of the aerosol-generating articles for inhalation by a user.

Aerosol-generating articles for use with the aerosol-generating device may be assembled using known methods and machinery.

For the avoidance of doubt, features described above in relation to one aspect of the invention may also be applied to other aspects of the invention.

The invention will be further described, by way of example only, with reference to the accompanying drawings in which:
Figures 1a and 1b show an aerosol-generating device according to a first embodiment of the invention at a temperature below the threshold temperature;
Figure 1c shows the aerosol-generating device according to the first embodiment of the invention shown in Figure 1b at a temperature above the threshold temperature;
Figure 2a shows an aerosol-generating device according to a second embodiment of the invention at a temperature below the threshold temperature;
Figure 2b shows the aerosol-generating device according to the second embodiment of the invention at a temperature above the threshold temperature;
Figure 3a shows an aerosol-generating device according to a third embodiment of the invention at a temperature below the threshold temperature; and
Figure 3b shows the aerosol-generating device according to the third embodiment of the invention at a temperature above the threshold temperature;

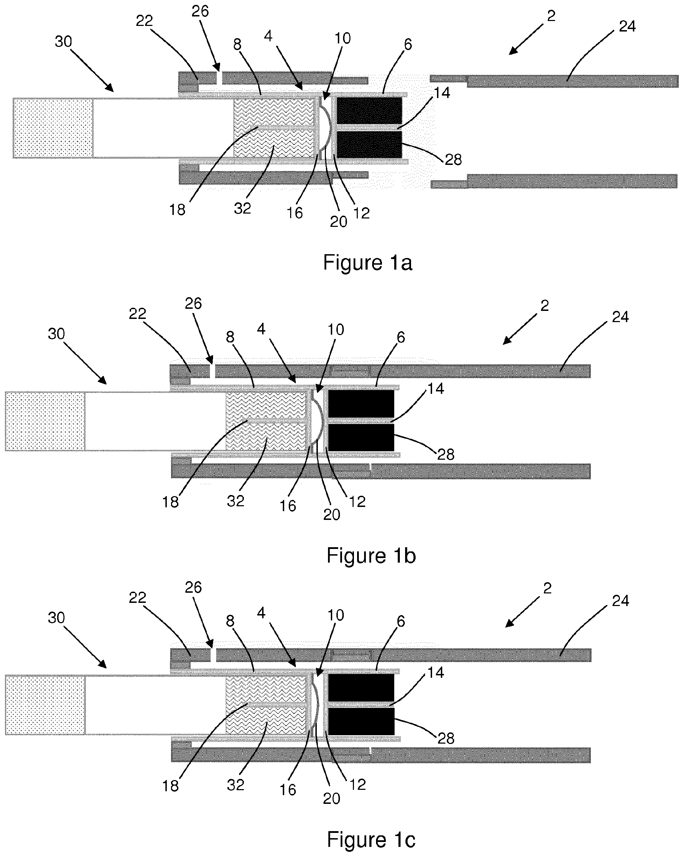

The aerosol-generating device 2 according to the first embodiment of the invention shown in Figures 1a to 1c comprises a housing 4 comprising a first rigid cylindrical tubular section 6 and a second rigid cylindrical tubular section 8. The first tubular section 6 and the second tubular section 8 of the housing 4 are formed of thermally-conductive material. The aerosol-generating device 2 comprises a heat-conductive element 10 disposed between the first tubular section 6 and the second tubular section 8 of the housing 4.

The heat-conductive element 10 comprises a first heat-conductive component proximate the first tubular section 6 of the housing 4. The first heat-conductive component comprises a first heat-conductive disk 12 and a first elongate heat-conductive member 14 that projects from the first heat-conductive disk 12 into the first tubular section 6 of the housing 4.

The heat-conductive element 10 also comprises a second heat-conductive component proximate the second tubular section 8 of the housing 4. The second heat-conductive component comprises a second heat-conductive disk 16 and a second elongate heat-conductive member 18 that projects from the second heat-conductive disk 16 into the second tubular section 8 of the housing 4.

The heat-conductive element 10 further comprises a bimetallic element in the form of a bimetallic strip 20 disposed between the first heat-conductive disk 12 of the first heat-conductive component and the second heat-conductive disk 16 of the second heat-conductive component. The opposed ends of the bimetallic strip 20 are secured to the second heat-conductive disk 16 of the second heat-conductive component.

The aerosol-generating device 2 further comprises a rigid heat-insulative cylindrical sleeve 22 around the second tubular section 8 of the housing 4 and a portion of the first tubular section 6 of the housing 4. The aerosol-generating device also comprises a rigid heat-insulative cylindrical cover 24 removably connected to the heat-insulative sleeve 22. As shown in Figures 1b and 1c, when connected to the heat-insulative sleeve 22, the heat-insulative cover 24 projects outwardly beyond the first tubular section 6 of the housing 4. One or more air inlets 26 are provided in the heat-insulative sleeve 22 around the second tubular section 8 of the housing 4.

To use the aerosol-generating device 2, the heat-insulative cover 24 is disconnected from the heat-insulative sleeve 22 as shown in Figure 1a. A solid cylindrical combustible carbonaceous heat source 28 comprising an axial cavity is inserted into the first tubular section 6 of the housing 4. The end face of the combustible heat source 28 abuts the first heat-conductive disk 12 of the first heat-conductive component of the heat-conductive element 10. The first elongate heat-conductive member 14 of the first heat-conductive component of the heat-conductive element 10 projects into the axial cavity of the combustible heat source 28.

An aerosol-generating article 30 comprising an aerosol-generating substrate 32 is inserted into the second tubular section 8 of the housing 4. The end face of the aerosol-generating substrate 32 of the aerosol-generating article 30 abuts the second heat-conductive disk 16 of the second heat-conductive component of the heat-conductive element 10. The second elongate heat-conductive member 18 of the second heat-conductive component of the heat-conductive element 10 projects into the aerosol-generating substrate 32 of the aerosol-generating article 30.

The combustible heat source 28 is ignited using a lighter or other suitable device and the heat-insulative cover 24 is then connected to the heat-insulative sleeve 22 as shown in Figure 1b.

Initially the bimetallic strip 20 of the heat conductive element 10 is in the first position shown in Figure 1b. In the first position the bimetallic strip 20 is convexly curved relative to the first tubular section 6 of the housing 4 and is in direct contact with the first heat-conductive disk 12 of the first heat-conductive component of the heat-conductive element 10.

Following ignition of the combustible heat source 28, the aerosol-generating substrate 32 of the aerosol-generating article 30 in the second tubular section 8 of the housing 4 is heated by conductive heat transfer from the combustible heat source 28 in the first tubular section 6 of the housing 4 via the first tubular section 6 and the second tubular section 8 of the housing 4 and via the heat-conductive element 10. Volatile compounds are released from the heated aerosol-generating substrate 32 and entrained in air drawn through the aerosol-generating article 30. As the released compounds cool, they condense to form an aerosol that is inhaled by the user through the mouth end of the aerosol-generating article 30.

When the bimetallic strip 20 of the heat-conductive element 10 is heated above a threshold temperature by the combustible heat source 28 it deforms from the first position shown in Figure 1b to the second position shown in Figure 1c. In the second position, the bimetallic strip 20 is no longer in direct contact with the first heat-conductive disk 12 of the first heat-conductive component of the heat-conductive element 10 and the aerosol-generating substrate 32 of the aerosol-generating article 30 in the second tubular portion 8 of the housing 4 is no longer heated by conductive heat transfer from the combustible heat source 28 in the first tubular section 6 of the housing 4 via the heat-conductive element 10.

In the second position, heat is transferred by conduction from the heat-conductive element 10 comprising the bimetallic strip 20 to the aerosol-generating substrate 32 of the aerosol-generating article 30 received in the second tubular section 8 of the housing 4 and the bimetallic strip 20 cools. When the bimetallic strip 20 cools below the threshold temperature, the bimetallic strip 20 deforms from the second position shown in Figure 1c to the first position shown in Figure 1b. In the first position, the bimetallic strip is once again in direct contact with the first heat-conductive disk 12 of the first heat-conductive component of the heat-conductive element 10 and the aerosol-generating substrate 32 of the aerosol-generating article 30 is once again heated by conductive heat transfer from the combustible heat source 28 in the first tubular section 6 of the housing 4 via the heat-conductive element 10.

Once one or both of the combustible heat source and the aerosol-generating substrate 32 of the aerosol-generating article 30 have been consumed, the heat-insulative cover 24 may be disconnected from the heat-insulative sleeve 22 as shown in Figure 1a. One or both of the combustible heat source 28 and the aerosol-generating article 30 may then be removed from the first tubular section 6 and the second tubular section 8 of the housing 4, respectively.

To reuse the aerosol-generating device 2, one or both of a further combustible heat source 28 and a further aerosol-generating article 30 may subsequently be inserted into the first tubular section 6 and the second tubular section 8 of the housing 4, respectively.

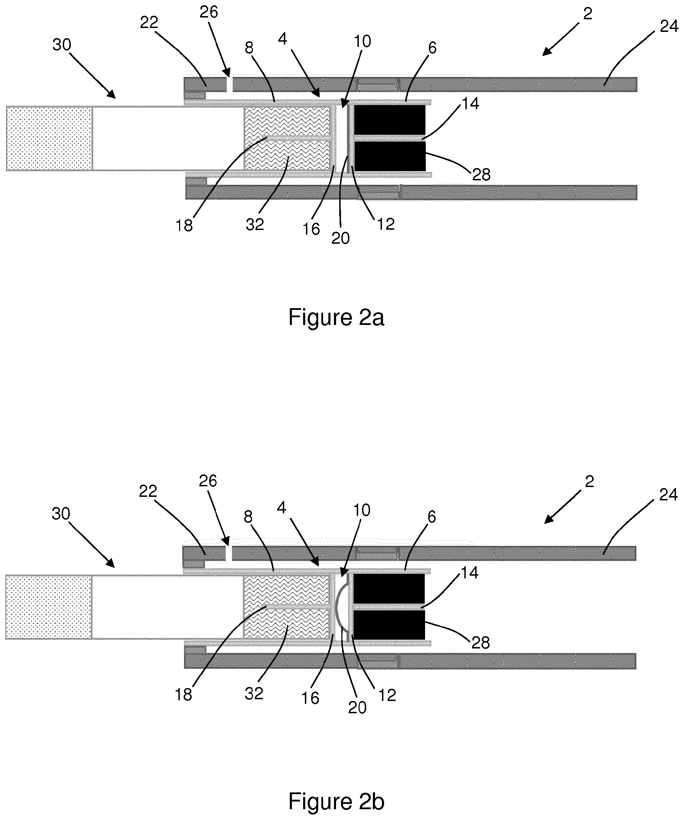

The aerosol-generating device 2 according to the second embodiment of the invention shown in Figures 2a and 2b is of largely similar construction to the aerosol-generating device according to the first embodiment of the invention shown in Figures 1a to 1c. However, in the aerosol-generating device according to the second embodiment of the invention the opposed ends of the bimetallic strip 20 of the heat conductive element 10 are secured to the first heat-conductive disk 12 of the first heat-conductive component.

Initially the bimetallic strip 20 of the heat conductive element 10 is in the first position shown in Figure 2a. In the first position the bimetallic strip 20 is flat and in direct contact with the first heat-conductive disk 12 of the first heat-conductive component of the heat-conductive element 10.

Following ignition of a combustible heat source 28 inserted into the first tubular section 6 of the housing 4, the aerosol-generating substrate 32 of an aerosol-generating article 30 inserted into the second tubular section of the housing 4 is heated by conductive heat transfer from the combustible heat source 28 in the first tubular section 6 of the housing 4 via the first tubular section 6 and the second tubular section 8 of the housing. In the first position, the bimetallic strip is not in direct contact with the second heat-conductive disk 16 of the first heat-conductive component of the heat-conductive element 10 and the aerosol-generating substrate 32 of the aerosol-generating article 30 is not heated by conductive heat transfer from the combustible heat source 28 in the first tubular section 6 of the housing 4 via the heat-conductive element 10.

Volatile compounds are released from the heated aerosol-generating substrate 32 and entrained in air drawn through the aerosol-generating article 30. As the released compounds cool, they condense to form an aerosol that is inhaled by the user through the mouth end of the aerosol-generating article 30.

When the bimetallic strip 20 of the heat-conductive element 10 is heated above a threshold temperature by the combustible heat source 28 it deforms from the first position shown in Figure 2a to the second position shown in Figure 2b. In the second position, the bimetallic strip is in direct contact with the second heat-conductive disk 16 of the second heat-conductive component of the heat-conductive element 10 and the aerosol-generating substrate 32 of the aerosol-generating article 30 is heated by conductive heat transfer from the combustible heat source 28 in the first tubular section 6 of the housing 4 via the heat-conductive element 10.

In the second position, the bimetallic strip 20 cools. When the bimetallic strip 20 cools below the threshold temperature, the bimetallic strip 20 deforms from the second position shown in Figure 2b to the first position shown in Figure 2a. In the first position, the bimetallic strip 20 is no longer in direct contact with the second heat-conductive disk 16 of the second heat-conductive component of the heat-conductive element 10 and the aerosol-generating substrate 32 of the aerosol-generating article 30 in the second tubular portion 8 of the housing 4 is no longer heated by conductive heat transfer from the combustible heat source 28 in the first tubular section 6 of the housing 4 via the heat-conductive element 10.

Once one or both of the combustible heat source and the aerosol-generating substrate 32 of the aerosol-generating article 30 have been consumed, the heat-insulative cover 24 may be disconnected from the heat-insulative sleeve 22. One or both of the combustible heat source 28 and the aerosol-generating article 30 may then be removed from the first tubular section 6 and the second tubular section 8 of the housing 4, respectively.

To reuse the aerosol-generating device 2, one or both of a further combustible heat source 28 and a further aerosol-generating article 30 may subsequently be inserted into the first tubular section 6 and the second tubular section 8 of the housing 4, respectively.

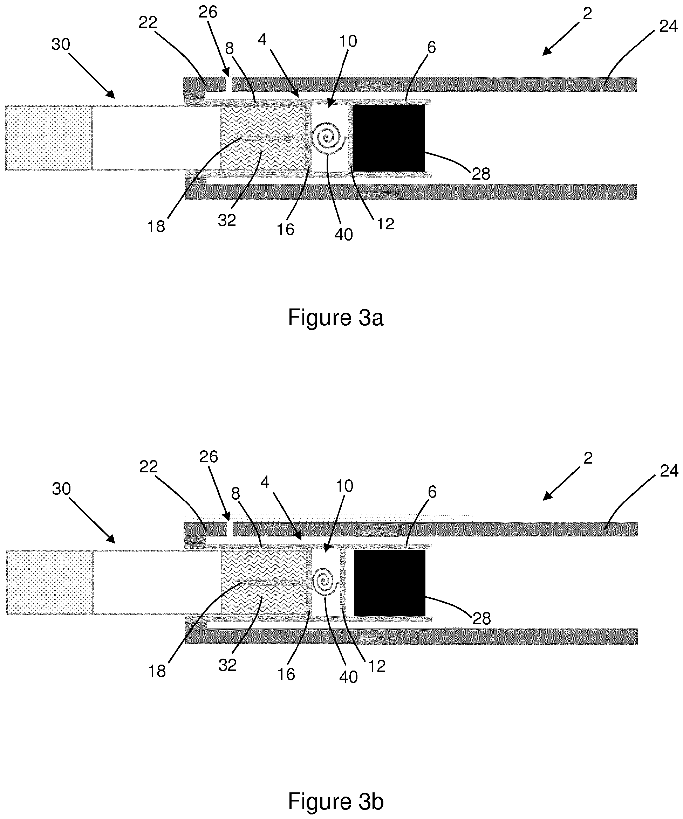

The aerosol-generating device 2 according to the third embodiment of the invention shown in Figures 3a and 3b is of similar construction to the aerosol-generating device according to the first embodiment of the invention shown in Figures 1a to 1c. However, in the aerosol-generating device 2 according to the third embodiment of the invention the bimetallic element is in the form of a bimetallic coil 40 and is secured to both the first heat-conductive disk 12 of the first heat-conductive component and the second heat-conductive disk 16 of the second heat-conductive component. In addition, in the aerosol-generating device according to the third embodiment of the invention the first elongate heat-conductive member 14 of the first heat-conductive component of the heat-conductive element 10 is omitted and the first heat-conductive disk 12 of the first heat-conductive component of the heat-conductive element 10 is movable relative to the first tubular section 6 of the housing 4.

Initially the bimetallic coil 40 of the heat conductive element 10 is in the first position shown in Figure 3a. In the first position the bimetallic coil 40 is loosely wound and the first heat-conductive disk 12 of the first heat-conductive component of the heat-conductive element 10 is in direct contact with a combustible heat source 28 inserted into the first tubular section 6 of the housing 4.

Following ignition of the combustible heat source 28, the aerosol-generating substrate 32 of an aerosol-generating article 30 inserted into the second tubular section 8 of the housing 4 is heated by conductive heat transfer from the combustible heat source 28 in the first tubular section 6 of the housing 4 via the first tubular section 6 and the second tubular section 8 of the housing 4 and via the heat-conductive element 10. Volatile compounds are released from the heated aerosol-generating substrate 32 and entrained in air drawn through the aerosol-generating article 30. As the released compounds cool, they condense to form an aerosol that is inhaled by the user through the mouth end of the aerosol-generating article 30.

When the bimetallic coil 40 of the heat-conductive element 10 is heated above a threshold temperature by the combustible heat source 28 it deforms from the first position shown in Figure 3a to the second position shown in Figure 3b. In the second position, the bimetallic coil 40 is tightly wound, the first heat-conductive disk 12 of the first heat-conductive component of the heat-conductive element 10 is no longer in direct contact with the combustible heat source 28 and the aerosol-generating substrate 32 of the aerosol-generating article 30 in the second tubular portion 8 of the housing 4 is no longer heated by conductive heat transfer from the combustible heat source 28 in the first tubular section 6 of the housing 4 via the heat-conductive element 10.

In the second position, heat is transferred by conduction from the heat-conductive element 10 comprising the bimetallic coil 40 to the aerosol-generating substrate 32 of the aerosol-generating article 30 received in the second tubular section 8 of the housing 4 and the bimetallic coil 40 cools. When the bimetallic coil 40 cools below the threshold temperature, the bimetallic coil 40 deforms from the second position shown in Figure 3b to the first position shown in Figure 3a. In the first position, the bimetallic strip is loosely wound, the first heat-conductive disk 12 of the first heat-conductive component of the heat-conductive element 10 is once again in direct contact with combustible heat source 28 and the aerosol-generating substrate 32 of the aerosol-generating article 30 is once again heated by conductive heat transfer from the combustible heat source 28 in the first tubular section 6 of the housing 4 via the heat-conductive element 10.

Once one or both of the combustible heat source and the aerosol-generating substrate 32 of the aerosol-generating article 30 have been consumed, the heat-insulative cover 24 of the aerosol-generating device 2 may be disconnected from the heat-insulative sleeve 22 thereof. One or both of the combustible heat source 28 and the aerosol-generating article 30 may then be removed from the first tubular section 6 and the second tubular section 8 of the housing 4, respectively.

To reuse the aerosol-generating device 2, one or both of a further combustible heat source 28 and a further aerosol-generating article 30 may subsequently be inserted into the first tubular section 6 and the second tubular section 8 of the housing 4, respectively.

In the aerosol-generating devices according to the first, second and third embodiments of the invention shown in Figures 1a to 1c, 2a and 2b and 3a and 3b, respectively, deformation of the bimetallic element 20 between the first position and the second position provides thermostatic control of the temperature of the aerosol-generating substrate 32 of the aerosol-generating article 30 received in the second tubular section 8 of the housing 4 through dynamic adjustment of the conductive heat transfer from the combustible heat source 28 received in the first tubular section 6 of the housing 4 to the aerosol-generating substrate 32 of the aerosol-generating 30 via the heat-conductive element. The bimetallic element 20 thereby maintains the temperature of the aerosol-generating substrate 32 of the aerosol-generating article 30 within a certain range in order to optimise the aerosol delivery to the user.

## Claims

1. An aerosol-generating device (2) comprising:
a housing (4) comprising a first tubular section (6) for receiving a combustible heat source (28) and a second tubular section (8) for receiving an aerosol-generating substrate (32);
a heat-conductive element (10) disposed between the first tubular section (6) and the second tubular section (8) of the housing (4) for transferring heat from a combustible heat source (28) received in the first tubular section (6) to an aerosol-generating substrate (32) received in the second tubular section (8), the heat-conductive element (10) comprising a bimetallic element (20) having a first portion and a second portion; and
a heat-insulative sleeve (22) around at least a portion of the second tubular section (8) of the housing (4), wherein the heat-insulative sleeve (22) comprises one or more air inlets (26),
wherein the bimetallic element (20) is arranged to deform from a first position, in which a first portion of the bimetallic element (20) is proximate the first tubular section (6) of the housing (4) and a second portion of the bimetallic element (20) is proximate the second tubular section (8) of the housing (4), to a second position, in which the first portion of the bimetallic element (20) is displaced away from the first tubular section (6) of the housing (4) towards the second tubular section (8) of the housing (4), when the bimetallic element (20) is heated above a threshold temperature.

2. An aerosol-generating device (2) according to claim 1 wherein the bimetallic element (20) is pre-stressed such that it deforms from the first position to the second position with a snap action.

3. An aerosol-generating device (2) according to claim 1 or 2 wherein the bimetallic element (20) bends from the first position to the second position when heated above the threshold temperature.

4. An aerosol-generating device (2) according to claim 3 wherein the bimetallic element (20) is flat or convex relative to the first tubular section (6) in the first position and concave relative to the first tubular section (6) in the second position.

5. An aerosol-generating device (2) according to any one of claims 1 to 4 wherein the heat-conductive element (10) further comprises a first elongate heat-conductive member (14) that projects into the first tubular section (6) of the housing (4) for insertion into a combustible heat source (28) received in the first tubular section (6).

6. An aerosol-generating device (2) according to claim 5 wherein the heat-conductive element (10) further comprises a second elongate heat-conductive member (18) that projects into the second tubular section (8) of the housing (4) for insertion into an aerosol-generating substrate (32) received in the second tubular section (8).

7. An aerosol-generating device (2) according to claim 1 or 2 wherein the bimetallic element (20) contracts from the first position to the second position when heated above the threshold temperature.

8. An aerosol-generating device (2) according to claim 7 wherein the bimetallic element (20) is a bimetallic helical coil or a bimetallic spiral coil.

9. An aerosol-generating device (2) according to any one of claims 1 to 6 wherein the housing (4) is configured such that airflow from the first tubular section (6) to the second tubular section (8) of the housing (4) is substantially prevented.

10. An aerosol-generating device (2) according to any one of claims 1 to 7 wherein one or more air inlets are provided in the second tubular section (8).

11. An aerosol-generating device (2) according to any one of claims 1 to 10 wherein one or both of the first tubular section (6) and the second tubular section (8) of the housing (4) comprise thermally-conductive material.

12. An aerosol-generating device (2) according to any one of claims 1 to 11 further comprising:
a heat-insulative cover (24) around at least a portion of the first tubular section (6) wherein the heat-insulative cover (24) is removably connected to the heat-insulative sleeve (22).

13. An aerosol-generating system comprising:
an aerosol-generating device (2) according to any one of claims 1 to 12; and
one or more combustible heat sources (28) for insertion into the first tubular section (6) of the aerosol-generating device (2).

## Patentansprüche

1. Aerosolerzeugungsvorrichtung (2), umfassend:
ein Gehäuse (4), umfassend einen ersten rohrförmigen Teilbereich (6) zum Aufnehmen einer brennbaren Wärmequelle (28) und einen zweiten rohrförmigen Teilbereich (8) zum Aufnehmen eines aerosolerzeugenden Substrats (32);
ein zwischen dem ersten rohrförmigen Teilbereich (6) und dem zweiten rohrförmigen Teilbereich (8) des Gehäuses (4) angeordnetes wärmeleitendes Element (10) zur Übertragung von Wärme von einer in dem ersten rohrförmigen Teilbereich (6) aufgenommenen brennbaren Wärmequelle (28) auf ein in dem zweiten rohrförmigen Teilbereich (8) aufgenommenes aerosolerzeugendes Substrat (32), wobei das wärmeleitende Element (10) ein Bimetallelement (20) mit einem ersten Abschnitt und einem zweiten Abschnitt aufweist; und
eine wärmeisolierende Hülse (22) um wenigstens einen Abschnitt des zweiten rohrförmigen Teilbereichs (8) des Gehäuses (4), wobei die wärmeisolierende Hülse (22) einen oder mehrere Lufteinlässe (26) aufweist,
wobei das Bimetallelement (20) zur Verformung aus einer ersten Position, in der sich ein erster Abschnitt des Bimetallelements (20) in der Nähe des ersten rohrförmigen Teilbereichs (6) des Gehäuses (4) und ein zweiter Abschnitt des Bimetallelements (20) in der Nähe des zweiten rohrförmigen Teilbereichs (8) des Gehäuses (4) befindet, in eine zweite Position, in der der erste Abschnitt des Bimetallelements (20) von dem ersten rohrförmigen Teilbereich (6) des Gehäuses (4) weg in Richtung des zweiten rohrförmigen Teilbereichs (8) des Gehäuses (4) verschoben wird, wenn das Bimetallelement (20) über eine Schwellentemperatur erwärmt wird, angeordnet ist.

2. Aerosolerzeugungsvorrichtung (2) nach Anspruch 1, wobei das Bimetallelement (20) vorgespannt ist, sodass es sich von der ersten Position zu der zweiten Position mit einer Sprungwirkung deformiert.

3. Aerosolerzeugungsvorrichtung (2) nach Anspruch 1 oder 2, wobei sich das Bimetallelement (20) von der ersten Position in die zweite Position biegt, wenn es über die Schwellentemperatur erwärmt wird.

4. Aerosolerzeugungsvorrichtung (2) nach Anspruch 3, wobei das Bimetallelement (20) in der ersten Position relativ zu dem ersten rohrförmigen Teilbereich (6) flach oder konvex und in der zweiten Position relativ zu dem ersten rohrförmigen Teilbereich (6) konkav ist.

5. Aerosolerzeugungsvorrichtung (2) nach einem der Ansprüche 1 bis 4, wobei das wärmeleitende Element (10) ferner ein erstes längliches wärmeleitendes Element (14) aufweist, das in den ersten rohrförmigen Teilbereich (6) des Gehäuses (4) vorsteht, um in eine in dem ersten rohrförmigen Teilbereich (6) aufgenommene brennbare Wärmequelle (28) eingesetzt zu werden.

6. Aerosolerzeugungsvorrichtung (2) nach Anspruch 5, wobei das wärmeleitende Element (10) ferner ein zweites längliches wärmeleitendes Element (18) aufweist, das in den zweiten rohrförmigen Teilbereich (8) des Gehäuses (4) vorsteht, um in ein aerosolerzeugendes Substrat (32) eingesetzt zu werden, das in den zweiten röhrenförmigen Teilbereich (8) aufgenommen ist.

7. Aerosolerzeugungsvorrichtung (2) nach Anspruch 1 oder 2, wobei sich das Bimetallelement (20) von der ersten Position in die zweite Position zusammenzieht, wenn es über die Schwellentemperatur erwärmt wird.

8. Aerosolerzeugungsvorrichtung (2) nach Anspruch 7, wobei das Bimetallelement (20) eine Bimetallwendelspule oder eine Bimetallspiralspule ist.

9. Aerosolerzeugungsvorrichtung (2) nach einem der Ansprüche 1 bis 6, wobei das Gehäuse (4) dahingehend ausgelegt ist, dass ein Luftstrom von dem ersten rohrförmigen Teilbereich (6) zu dem zweiten rohrförmigen Teilbereich (8) des Gehäuses (4) im Wesentlichen verhindert wird.

10. Aerosolerzeugungsvorrichtung (2) nach einem der Ansprüche 1 bis 7, wobei ein oder mehrere Lufteinlässe in dem zweiten rohrförmigen Teilbereich (8) vorgesehen sind.

11. Aerosolerzeugungsvorrichtung (2) nach einem der Ansprüche 1 bis 10, wobei der erste rohrförmige Teilbereich (6) und/oder der zweite rohrförmige Teilbereich (8) des Gehäuses (4) wärmeleitendes Material umfassen.

12. Aerosolerzeugungsvorrichtung (2) nach einem der Ansprüche 1 bis 11, weiter aufweisend:
eine wärmeisolierende Abdeckung (24) um wenigstens einen Abschnitt des ersten rohrförmigen Teilbereichs (6), wobei die wärmeisolierende Abdeckung (24) entfernbar mit der wärmeisolierenden Hülse (22) verbunden ist.

13. Aerosolerzeugungssystem, aufweisend:
eine Aerosolerzeugungsvorrichtung (2) nach einem beliebigen der Ansprüche 1 bis 12; und
eine oder mehrere brennbare Wärmequellen (28) zum Einsetzen in den ersten rohrförmigen Teilbereich (6) der Aerosolerzeugungsvorrichtung (2).

## Revendications

1. Dispositif de génération d'aérosol (2) comprenant :
un logement (4) comprenant une première section tubulaire (6) destinée à recevoir une source de chaleur combustible (28) et une deuxième section tubulaire (8) destinée à recevoir un substrat de génération d'aérosol (32) ;
un élément thermoconducteur (10) disposé entre la première section tubulaire (6) et la deuxième section tubulaire (8) du logement (4) destiné à transférer la chaleur d'une source de chaleur combustible (28) reçue dans la première section tubulaire (6) vers un substrat de génération d'aérosol (32) reçu dans la deuxième section tubulaire (8), l'élément thermoconducteur (10) comprenant un élément bimétallique (20) ayant une première partie et une deuxième partie ; et
un manchon calorifuge (22) autour d'au moins une partie de la deuxième section tubulaire (8) du logement (4), dans lequel le manchon calorifuge (22) comprend une ou plusieurs entrées d'air (26),
dans lequel l'élément bimétallique (20) est agencé pour se déformer à partir d'une première position, dans laquelle une première partie de l'élément bimétallique (20) est à proximité de la première section tubulaire (6) du logement (4) et une deuxième partie de l'élément bimétallique (20) est à proximité de la deuxième section tubulaire (8) du logement (4), jusqu'à une deuxième position, dans laquelle la première partie de l'élément bimétallique (20) est déplacée à l'écart de la première section tubulaire (6) du logement (4) vers la deuxième section tubulaire (8) du logement (4), lorsque l'élément bimétallique (20) est chauffé au-dessus d'une température seuil.

2. Dispositif de génération d'aérosol (2) selon la revendication 1, dans lequel l'élément bimétallique (20) est précontraint de sorte qu'il se déforme à partir de la première position jusqu'à la deuxième position avec une action de déclic.

3. Dispositif de génération d'aérosol (2) selon la revendication 1 ou 2, dans lequel l'élément bimétallique (20) se courbe de la première position vers la deuxième position lorsqu'il est chauffé au-dessus de la température seuil.

4. Dispositif de génération d'aérosol (2) selon la revendication 3, dans lequel l'élément bimétallique (20) est plat ou convexe par rapport à la première section tubulaire (6) dans la première position et concave par rapport à la première section tubulaire (6) dans la deuxième position.

5. Dispositif de génération d'aérosol (2) selon l'une quelconque des revendications 1 à 4, dans lequel l'élément thermoconducteur (10) comprend en outre un premier membre allongé thermoconducteur (14) qui fait saillie dans la première section tubulaire (6) du logement (4) pour insertion dans une source de chaleur combustible (28) reçue dans la première section tubulaire (6).

6. Dispositif de génération d'aérosol (2) selon la revendication 5, dans lequel l'élément thermoconducteur (10) comprend en outre un deuxième membre allongé thermoconducteur (18) qui fait saillie dans la deuxième section tubulaire (8) du logement (4) pour insertion dans un substrat de génération d'aérosol (32) reçu dans la deuxième section tubulaire (8).

7. Dispositif de génération d'aérosol (2) selon la revendication 1 ou 2, dans lequel l'élément bimétallique (20) se contracte de la première position vers la deuxième position lorsqu'il est chauffé au-dessus de la température seuil.

8. Dispositif de génération d'aérosol (2) selon la revendication 7, dans lequel l'élément bimétallique (20) est une bobine hélicoïdale bimétallique ou une bobine en spirale bimétallique.

9. Dispositif de génération d'aérosol (2) selon l'une quelconque des revendications 1 à 6, dans lequel le logement (4) est configuré de telle sorte qu'un écoulement d'air depuis la première section tubulaire (6) vers la deuxième section tubulaire (8) du logement (4) est sensiblement empêché.

10. Dispositif de génération d'aérosol (2) selon l'une quelconque des revendications 1 à 7, dans lequel une ou plusieurs entrées d'air sont prévues dans la deuxième section tubulaire (8).

11. Dispositif de génération d'aérosol (2) selon l'une quelconque des revendications 1 à 10, dans lequel l'une ou les deux de la première section tubulaire (6) et de la deuxième section tubulaire (8) du logement (4) comprennent un matériau thermoconducteur.

12. Dispositif de génération d'aérosol (2) selon l'une quelconque des revendications 1 à 11, comprenant en outre :
une enveloppe calorifuge (24) autour d'au moins une partie de la première section tubulaire (6) dans lequel l'enveloppe calorifuge (24) est raccordée de manière amovible au manchon calorifuge (22).

13. Système de génération d'aérosol comprenant :
un dispositif de génération d'aérosol (2) selon l'une quelconque des revendications 1 à 12 ; et
une ou plusieurs sources de chaleur combustibles (28) pour insertion dans la première section tubulaire (6) du dispositif de génération d'aérosol (2).
